# EUROPEAN PATENT APPLICATION

(11) **EP 1 484 612 A1**
(43) Date of publication of application: **08.12.2004**
(21) Application number: 04011660.0
(22) Date of filing: 17.05.2004
(51) Int. Cl.: G01N 33/46

(54) **Method and apparatus for identifying foreign objects within wood-like material**

(30) Priority: 26.05.2003 IT MO20030156
(71) Applicant: Benedetti, Paolo, 41100 Modena (IT)
(72) Inventor: Benedetti, Paolo, 41100 Modena (IT)
(74) Representative: Modiano, Guido, Dr.-Ing.

(57) **Abstract**

A method for identifying foreign objects within a stream of material, particularly substantially uniform wood-like material, comprising the steps of striking a stream of material with a beam of electromagnetic waves of known intensity that are oriented substantially transversely to the direction of advancement of the stream, detecting the intensity of the wave beam downstream of the stream of material, and comparing the intensities of the wave beam upstream and downstream of the stream of material, the absorption coefficient of the portion of stream that is crossed being substantially proportional to the difference between the intensities of the wave beam upstream and downstream of the portion.

## Description

The present invention relates to a method for identifying foreign objects within a stream of material, particularly substantially uniform wood-like material, and to the corresponding apparatus.

It is known that in recent years there has been widespread industrial use of panels obtained by pressing loose wood-like materials, such as fibers, chips or shavings, obtained starting from wood products, generally recovered from landfills or from waste of industrial processes.

These panels are used for example in the fields of furniture, building, and vehicle and watercraft manufacture.

Raw materials for manufacturing such panels are fed to the equipment provided along the lines for producing the panels, such as mills or presses, by way of conventional conveyance system of the continuous or gravity type.

It is also known that these materials are often mixed in with foreign objects, constituted for example by lumps of glue, pieces of plastics, metallic residues or stones, which can give rise to various drawbacks during the production process for obtaining the panels and during the subsequent processing of said panels.

For example, the presence of such foreign objects can cause malfunctions in the equipment provided along the production lines with the consequent need to suspend production in order to restore the operation of said equipment.

Moreover, particularly if it is necessary to provide thin panels, with thicknesses on the order of a few millimeters, the presence of the foreign objects produces a high percentage of defective panels and therefore of rejects and can cause serious damage to the belts or calibrated surfaces of the presses, which therefore have to be replaced in order to ensure the production of panels that are free from surface defects.

Finally, in the subsequent processing of the panels the presence of foreign objects can damage, for example, the tools used in cutting machines, which become unusable if they make contact with metallic or stone-like elements and therefore need to be replaced.

Devices that use an electromagnetic field that passes through the stream of material, such as so-called metal detectors, are currently used to detect any metallic bodies contained within the stream of wood-like material.

However, even the use of these devices has limitations in use, mainly due to the high speed at which the material advances along the production lines and to the need to perform continuous detection.

Identification systems that can be applied along the stream of material and in particular upstream of the mills or presses are instead not known for foreign objects of other materials.

The aim of the present invention is to eliminate the drawbacks cited above of the known art, by providing a method for identifying foreign objects within a stream of material, particularly substantially uniform wood-like material, and a corresponding apparatus, which allows to identify any type of foreign object that is present in the stream of raw materials that feed the machines of the lines for producing pressed panels, regardless of the nature of the material that constitutes said foreign objects, and to improve the quality of the resulting panels and avoid damage to the equipment.

Within this aim, an object of the present invention is to provide a method that is simple, relatively easy to provide in practice, safe in use, effective in operation, and has a relatively low cost.

This aim and this and other objects that will become better apparent hereinafter are achieved by the present method for identifying foreign objects within a stream of material, particularly substantially uniform wood-like material, characterized in that it comprises the steps of striking a stream of material with a beam of electromagnetic waves that are oriented substantially transversely to the direction of advancement of said stream, detecting intensity of said wave beam downstream of said stream of material, and comparing the intensities of said wave beam upstream and downstream of said stream of material, the absorption coefficient of the portion of stream that is crossed being substantially proportional to the difference between the intensities of the wave beam upstream and downstream of said portion.

The corresponding apparatus is characterized in that it comprises means for emitting said wave beam which are arranged at a surface for the advancement of said stream of material, means for receiving and detecting the intensity of said wave beam downstream of said stream of material, which cooperate with said emission means and are arranged on the opposite side of said surface with respect to said emission means, and a processing unit, which is functionally associated with said emission means and said reception and detection means and is suitable to compare the intensities of said wave beam upstream and downstream of said stream of material.

Further characteristics and advantages of the present invention will become better apparent from the following detailed description of a preferred but not exclusive embodiment of a method for identifying foreign objects within a stream of material, particularly substantially uniform wood-like material, and of the corresponding apparatus, illustrated by way of nonlimiting example in the accompanying drawings, wherein:
Figure 1 is a schematic view of a possible application of the method according to the invention;
Figure 2 is a schematic view of another application of the method according to the invention.

With reference to the figures, the reference numeral 1 generally designates an apparatus for identifying foreign objects within a stream of material, particularly substantially uniform wood-like material.

The apparatus 1 can be applied along the production lines of panels obtained by pressing wood fibers, chips or dust and in particular upstream of equipment such as presses and mills.

The apparatus 1 allows to detect, within a stream of uniform material, the presence of foreign objects of another kind; in a preferred embodiment, the apparatus 1 can be applied to a conventional system for transferring loose wood-like material along a line for manufacturing pressed panels in order to allow identification and subsequent separation of objects made of plastics, metal, stone or any material that is not wood-like.

The apparatus 1 comprises means 2 for emitting a beam 3 of electromagnetic waves 4 of known intensity that lie transversely to a stream of material to be analyzed, said means being arranged at a surface 5 for the advancement of the stream of material along an advancement direction, designated by the letter A in the figures.

The emission means 2 are preferably constituted by one or more conventional X-ray emission tubes distributed along the width of the stream of material.

Conveniently, the emission tubes are distributed over the entire width of the stream of material.

The emission means 2 are associated with corresponding electric power supply means, which are not shown in the figures because they are of a known type.

The apparatus 1 can be provided with a conventional filtering device of the bandpass type, not shown in the figures, which is associated with the emission means 2 and is suitable to select the waves 4 of a chosen frequency.

It is also possible to provide a so-called collimator, not shown in the figures, which is suitable to collimate the waves 4 produced by the emission means 2 and optionally filtered, so as to obtain a sort of curtain of waves 4 that are mutually parallel and propagate at right angles to the surface 5.

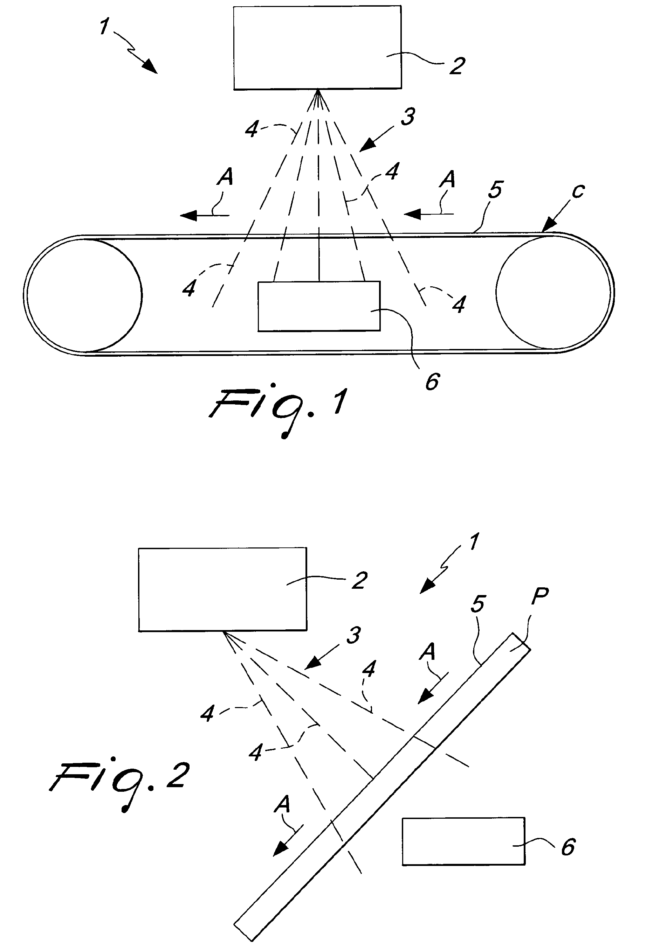

The surface 5 can be formed for example by a conventional continuous conveyor C (Figure 1), such as a conveyor belt, or by a surface P for the sliding of the material by gravity (Figure 2).

The apparatus 1 further comprises means 6 for receiving and detecting the intensity of the waves 4 downstream of the stream of material, which cooperate with the emission means 2 and are arranged on the opposite side of the surface 5 with respect to the emission means 2.

The reception and detection means 6 are preferably constituted by one or more diodes, which have a suitable resolution and are distributed along the width of the stream of material at said X-ray emission tubes.

Conveniently, said diodes are distributed along the entire width of the stream of material.

The emission means 2 and the reception and detection means 6 are preferably equipped with corresponding cooling means, for example of the oil- or air-based type.

In the illustrated embodiments, the emission means 2 and the reception and detection means 6 are arranged respectively above and below the surface 5; however, alternative embodiments of the apparatus 1, in which the arrangement of said means is reversed, are not excluded.

The machine 1 further has a processing unit, not shown in the figures, which is functionally associated with the emission means 2 and with the reception and detection means 6 and is suitable to compare the difference of the intensities of the waves 4 downstream and upstream of the stream of material with a set threshold value.

The absorption coefficient of the material crossed by the waves 4 is an indicator of the nature of such material and is proportional to said difference.

The processing unit is typically constituted by a conventional microprocessor system.

The processing unit is provided with conventional means for interfacing with the power supply means of the emission means 2, so as to adjust the electric power with which the emission means are supplied.

Merely by way of example, it has been found that if the apparatus 1 is applied to the analysis of a stream of wood-like material and the emission means 2 generate waves 4 with an intensity of approximately 10 V, the reception and detection means 6 receive a signal on the order of magnitude of 9 V if the waves 4 do not cross any material, or on the order of magnitude of 1.5-7 V, depending on the thickness of the material that is crossed, if the waves 4 pass through wood-like material.

If the waves 4 pass for example through metallic or stone-like material, the reception and detection means 6 instead receive a signal whose intensity is lower than 1 V.

Conveniently, the apparatus 1 is provided with means, not shown in the figures, for signaling the presence, within the stream of analyzed material, of a foreign object, i.e., of an object constituted by a material whose nature is different from that of said stream.

Said signaling means are functionally associated with the processing unit and are activated by said unit when the difference between the intensities of the waves 4 upstream and downstream of the stream of material is substantially greater than the set threshold value.

The signaling means can be for example of the optical or acoustic type.

The optical signaling means can be constituted for example by a light-emitting diode (LED) or more preferably by a bar of LEDs, each of which is associated with a given portion of the stream of material.

The processing unit can be provided with conventional means for interfacing with an operator, with a computerized network for supporting a company management system, for example to perform statistical research, or to an automatic device for removing any detected foreign objects, which can be configured in various manners depending on the architecture of the line to which the apparatus 1 is applied.

The method according to the invention substantially comprises the steps of: striking the entire width of a stream of material with a beam 3 of electromagnetic waves 4, preferably X-rays, having a known intensity and arranged transversely to the direction of advancement A of said stream, detecting the intensity of the waves 4 downstream of the stream of material, comparing the intensities of the waves 4 upstream and downstream of the stream, and signaling a difference between the intensities that is at least equal to a preset threshold value.

In practice it has been found that the described invention achieves the intended aim and object.

The invention thus conceived is susceptible of numerous modifications and variations, all of which are within the scope of the appended claims.

All the details may further be replaced with other technically equivalent ones.

In practice, the materials used, as well as the shapes and dimensions, may be any according to requirements without thereby abandoning the scope of the protection of the appended claims.

The disclosures in Italian Patent Application No. MO2003A000156 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A method for identifying foreign objects within a stream of material, particularly substantially uniform wood-like material, **characterized in that** it comprises the steps of striking a stream of material with a beam of electromagnetic waves that are oriented substantially transversely to the direction of advancement of said stream, detecting the intensity of said wave beam downstream of said stream of material, and comparing the intensities of said wave beam upstream and downstream of said stream of material, the absorption coefficient of the portion of stream that is crossed being substantially proportional to the difference between the intensities of the wave beam upstream and downstream of said portion.

2. The method according to claim 1, **characterized in that** it comprises the step of signaling a difference between the intensities of said wave beam upstream and downstream of said stream of material that is at least equal to a preset threshold value.

3. The method according to one or more of the preceding claims, **characterized in that** said striking action consists in affecting the entire width of said stream of material with said wave beam.

4. The method according to one or more of the preceding claims, **characterized in that** said wave beam is constituted by a plurality of X-rays.

5. The method according to one or more of the preceding claims, **characterized in that** said waves are substantially parallel to each other.

6. The method according to one or more of the preceding claims, **characterized in that** said waves are substantially perpendicular to said direction of advancement.

7. An apparatus for performing the method according to one or more of the preceding claims, **characterized in that** it comprises means for emitting a wave beam which are arranged at a surface for the advancement of a stream of material, means for receiving and detecting the intensity of said wave beam downstream of said stream of material, which cooperate with said emission means and are arranged on the opposite side of said surface with respect to said emission means, and a processing unit, which is functionally associated with said emission means and with said reception and detection means and is suitable to compare the intensities of said wave beam upstream and downstream of said stream of material.

8. The apparatus according to claim 7, **characterized in that** said processing unit compares the difference of the intensities of said wave beam upstream and downstream of said stream of material with said preset threshold value.

9. The apparatus according to claim 7, **characterized in that** said emission means comprise at least one X-ray emission tube.

10. The apparatus according to claim 7, **characterized in that** said emission means comprise a plurality of emission tubes distributed along the width of said stream of material.

11. The apparatus according to claim 7, **characterized in that** it comprises at least one filtering device associated with said emission means.

12. The apparatus according to claim 7, **characterized in that** it comprises at least one collimator that is associated with said emission means.

13. The apparatus according to claim 7, **characterized in that** said reception and detection means comprise at least one diode.

14. The apparatus according to claim 7, **characterized in that** said reception and detection means comprise a plurality of diodes distributed along the width of said stream of material.

15. The apparatus according to claim 7, **characterized in that** it comprises means for signaling the presence of at least one foreign object within said stream of material, which are functionally associated with said processing unit and are activated by said unit when the difference between the intensities of said wave beam upstream and downstream of said stream of material is at least equal to said threshold value.

16. The apparatus according to claim 15, **characterized in that** said signaling means are of the optical or acoustic type.

17. The apparatus according to claim 7, **characterized in that** it comprises cooling means, which are associated with said emission means or said reception and detection means or with both.

18. The apparatus according to claim 17, **characterized in that** said cooling means are of the air- or oil-based type or the like.

19. The apparatus according to claim 7, **characterized in that** said processing unit is a microprocessor system.

20. The apparatus according to claim 7, **characterized in that** said processing unit is provided with means for interfacing with an operator.

21. The apparatus according to claim 7, **characterized in that** said processing unit is provided with means for interfacing with a computer network.

22. The apparatus according to claim 7, **characterized in that** said processing unit is provided with means for interfacing with an automatic device for removing said foreign object.

23. The apparatus according to claim 7, **characterized in that** it comprises means for the supply of electric power to said emission means.

24. The apparatus according to claim 23, **characterized in that** said processing unit comprises means for interfacing with said power supply means.

25. The apparatus according to claim 7, **characterized in that** said advancement surface is constituted by a continuous conveyor, a sliding surface, or both.
